# EUROPEAN PATENT SPECIFICATION

(11) **EP 4 344 408 B1**
(45) Date of publication and mention of the grant of the patent: **11.11.2026**
(21) Application number: 22747895.5
(22) Date of filing: 05.07.2022
(51) Int. Cl.: A61F 2/46, A61F 2/36

(54) **IMPLANT EXTRACTOR**
IMPLANTATEXTRAKTOR
EXTRACTEUR D'IMPLANTS

(30) Priority: 02.07.2021 US 202163217938 P
(43) Date of publication of application: 03.04.2024
(73) Proprietor: Shukla Medical Inc., St. Petersburg, FL 33709 (US)
(72) Inventor: KEACH, Nicholas Christopher, Lutz, FL 33559 (US)
(74) Representative: Swea IP Law AB
(86) International application number: PCT/US2022/036149
(87) International publication number: WO 2023/278898

(56) References cited:
- US-A- 4 993 410
- US-A1- 2003 220 698
- US-A1- 2006 200 162
- US-A1- 2016 059 403
- US-A1- 2016 067 057
- US-A1- 2020 121 474
- US-B2- 10 398 569

## Description

This invention relates to a surgical device for use in implant extraction such as the extraction of implanted hip stem implants and prostheses.

### BACKGROUND

Surgeons use implant extracting devices as tools to assist with the removal of implanted surgical implants such as hip stems and prostheses. These devices allow a surgeon to use the tool to clamp onto a portion of the implant, such as a trunnion or the like, and then apply forces to the tool to extract the implant.
US2003220698A1 discloses a rasp and rasp handle 300, comprising a rasp handle body having a rasp handle proximal end 302, a rasp handle distal end 204, and a rasp handle docking aperture 326 formed in said distal end and a locking jaw 306 moveable connected to said rasp handle body, said locking jaw moveable between a locked position in which post 318 is retained within said rasp handle locking aperture and a release position in which said post is not retained within said rasp handle locking aperture. The post may include a notch 320, said notch engaged by said locking jaw in the locked position.
US10398569B2 discloses an impactor-extractor for inserting or extracting a stem of a prosthesis into or from a bone can include a body having a first end and a second end. The body can include an impact head located at the first end. A lever can be connected to the body and pivotable about a first pivot point distal to the first end of the body. A base can be located at the second end of the body and can include a first taper connector sized to mate with a second taper connector of the stem. A lock can be located at and pivotable about a second pivot point proximate the second end and can include an extension configured to secure the stem to the first taper connector. Linkages can connect the lever and the lock. The impactor-extractor has a linkage comprising elements 107, 108 and 110. The combined link and jaw element 110 is pivotably mounted to the housing 104 and engages the surgical implant 116 with a pivoting motion.
US2020121474A1 discloses a system comprising handles and attachments that facilitate the removal of a prosthetic implant. The system comprises an implant stem grabber attachment 2001, which is connectable to an unillustrated slap hammer at threaded connection 2003 and which is adapted to engage a stem 2080 of an implant. The teeth 2006 of the stem grabber are tightened with the ball nose driver 2008 that torques the U-joint 2009 to drive the lower U-joint 2010 which rotates to translate the lower Femoral Hip grabber block 2006 to lock onto the stem neck 2080 against the front lip of the stem grabber 2020.
US 2016/067057 discloses an implant extractor for operably engaging a surgical implant, the implant extractor having a housing with a proximal end configured for connection to an extraction tool.
US 2006/200162 discloses a device for implanting a femoral component including a base assembly, a handle slidably attached to base assembly, a locking member slidably coupled to handle, and a locking actuator. Locking member has a locking end extending from handle, through an opening in base assembly and through notch in implant.

US 2016/059403 A1 discloses a removable handle for a surgical tool, in particular a femoral rasp. The handle comprises an internal recess accommodating locking means. Said locking means comprise a rod-like member and several pivotally connected links forming a toggle mechanism. Actuation of the locking means causes controlled axial sliding of a jaw element relative to the distal end of the housing.

### BRIEF SUMMARY

The invention is set out in the appended set of claims.
Despite the existence of conventional implant extracting devices, there remains a need for an implant extracting device that allows for easy removal and replacement of the implant engaging components that can wear after repeated use. Moreover, there remains a need for an implant extracting device that provides a movable implant engaging jaw that remains in a desired commanded position even when no external forces beside gravity is acting on the jaw. In addition, there remains a need for a shorter implant extracting device than those currently offered without compromising the function of the tool. These and other benefits of the invention are described in greater detail herein.

In one exemplary embodiment, an implant extracting tool is disclosed that has an elongate housing with a proximal end configured for connection to an extraction tool, a distal end having an opening for operably engaging the implant, and an elongate recess therein with a slot extending from the elongate recess to the opening. A jaw is slidably received within the slot and operably extending into the opening. A linkage is secured and received within the elongate recess of the housing. The linkage is pivotally secured to the jaw such that operation of the linkage slides the jaw within the slot towards and away from the opening.

Other disclosed embodiments can include an insert with teeth for engaging the implant operably secured to the extracting tool. The insert and jaw can be detachably secured to the implant extracting tool and easily removed and replaced as needed. The jaw is detachably secured within the elongate housing. Also, a lock mechanism can be provided to hold the extracting tool in a locked position when clamped onto an implant.

An implant extractor comprising: an elongate housing having: a proximal end configured for connection to an extraction tool, a distal end for operably engaging an implant, and an elongate recess extending therein; a jaw slidably received within the elongate recess and extending to the distal end of the elongate housing; a linkage assembly within the elongate recess of the housing, the linkage assembly including a linkage pivotally secured to the jaw such that operation of the linkage assembly slides the jaw towards and away from the distal end.

The elongate housing includes an opening at the distal end configured for receiving an implant. The elongate recess is in fluid communication with the opening.

The linkage assembly includes: a rod assembly; a proximal link operatively connected to the rod assembly; a proximal end of the rod assembly pivotably connected to a distal end of the proximal link; an intermediate link operatively connected to the rod assembly; and a bell link operatively connected to the intermediate link and the jaw, wherein placement of the handle in a first position causes the jaw to disengage a trunnion of a hip stem implant, and placement of the handle in a second position causes the jaw to engage the trunnion of the hip stem implant.

The implant extractor further comprising a handle pivotably connected to the housing and operatively engaged with the proximal link. The implant extractor further comprising a handle pivotably connected to the housing and operatively engaged with the linkage assembly. The placement of the handle in a first position causes the jaw to move to a disengagement state, and placement of the handle in a second position causes the jaw to move to an engagement state.

The rod assembly is a telescopic rod assembly. The rod assembly comprises: a proximal link connector operatively connected to the proximal link; an intermediate link connector operatively connected to the intermediate link; and a rotatable shaft rotatable about the proximal link connector and operatively engage with the intermediate link connector.

The implant extractor further comprising a dial operatively connected to the central rotatable shaft, whereby rotation of the dial in a first direction operates to unthread the intermediate link connector from the central rotatable shaft, and whereby rotation of the dial in a second direction opposite the first direction operates to thread the intermediate link connector into the central rotatable shaft.

The implant extractor further comprising a jaw link coupling the bell link to the jaw. The implant extractor further comprising a locking key operatively connected to the proximal link and moveable between an unlocked position and a locked position. The locking key locks the position of the handle in a fixed position. The locking key locks the position of the proximal link in a fixed position.

The jaw is detachably secured within the elongate housing. The implant extractor further including a removable insert detachably secured to the housing at a distal end, the removable insert having a surface for engaging an implant and an opening for receiving the jaw therethrough.

The implant extractor further comprising a cam lock configured to lock the linkage assembly in a fixed position. The implant extractor further comprising a cam lock configured to apply a locking force and lock the linkage assembly in a fixed position.

Other features and advantages of the subject disclosure will be apparent from the following more detailed description of the exemplary embodiments, taken in conjunction with the accompanying drawings which illustrate, by way of example, the principles of the subject disclosure.

### BRIEF DESCRIPTION OF THE SEVERAL VIEWS OF THE DRAWINGS

The foregoing summary, as well as the following detailed description of the exemplary embodiments of the subject disclosure, will be better understood when read in conjunction with the appended drawings. For the purpose of illustrating the subject disclosure, there are shown in the drawings exemplary embodiments. It should be understood, however, that the invention is not limited to the precise arrangements and instrumentalities shown.

In the drawings:
FIG. 1 is a distal perspective view of a hip stem implant extractor in accordance with an exemplary embodiment of the subject disclosure;
FIG. 2 is a left side view of the hip stem implant extractor of FIG. 1.
FIG. 3 is a right side view of the hip stem implant extractor of FIG. 1.
FIG. 4 is a posterior view of the hip stem implant extractor of FIG. 1.
FIG. 5 is an anterior view of the hip stem implant extractor of FIG. 1.
FIG. 6 is a top view of the hip stem implant extractor of FIG. 1.
FIG. 7 is a bottom view of the hip stem implant extractor of FIG. 1.
FIG. 8 is an exploded perspective view of the hip stem implant extractor of FIG. 1.
FIG. 9 is a perspective longitudinal cross-sectional view of the hip stem implant extractor of FIG. 1.
FIG. 10 is a longitudinal cross-sectional view of the hip stem implant extractor of FIG. 1.
FIG. 11 is an exploded view of the hip stem implant extractor of FIG. 1.
FIG. 12 is a distal perspective view of a linkage assembly of the hip stem implant extractor of FIG. 1 in accordance with an exemplary embodiment of the subject disclosure.
FIG. 13 is an enlarged, partial, perspective view of a distal end of the hip stem implant extractor of FIG. 1.
FIG. 14 is an enlarged perspective view of a jaw of the hip stem implant extractor of FIG. 1.
FIG. 15 is a partial enlarged isometric view of a central portion of the hip stem implant extractor of FIG. 1 with the external housing shown as transparent for purposes of illustrating the internal components of the implant extractor.
FIG. 16 is a left, cross-sectional view of a central portion of the hip stem implant extractor of FIG. 1.
FIG. 17 is a partial, left, perspective view of the hip stem implant extractor of FIG. 1, with the handle in an unlocked position.
FIG. 18 is a partial, left, perspective view of the hip stem implant extractor of FIG. 1 with the handle in a locked position.
FIG. 19 is an enlarged, cross-sectional view of a central portion of the hip stem implant extractor of FIG. 17 in the unlocked position.
FIG. 20 is an enlarged, cross-section al view of the central portion of the hip stem implant extractor of FIG. 18 in the locked position.
FIGS. 22-28 are various views of an implant extractor design in accordance with the subject disclosure.

### DETAILED DESCRIPTION

Reference will now be made in detail to the various embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments as long as such combinations fall within the scope of the appended claims. One skilled in the relevant art will recognize, in light of the description herein, that the present disclosure can be practiced without one or more of the specific features or advantages of a particular exemplary embodiment. In other instances, additional features and advantages may be recognized in certain embodiments that may not be present in all exemplary embodiments of the subject disclosure.

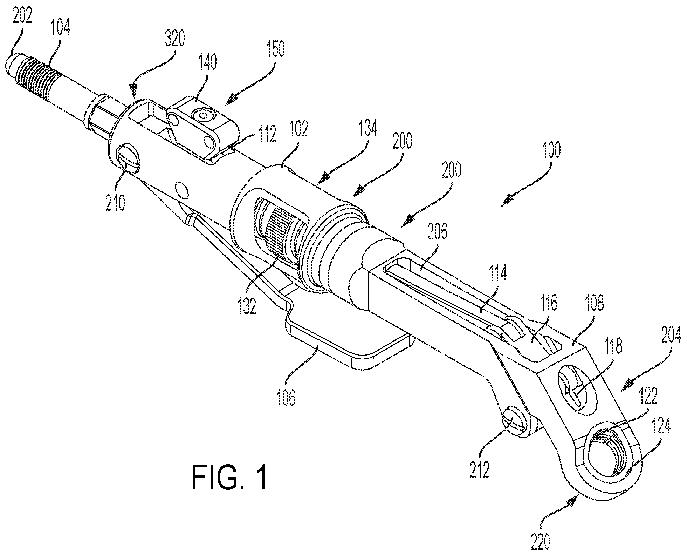

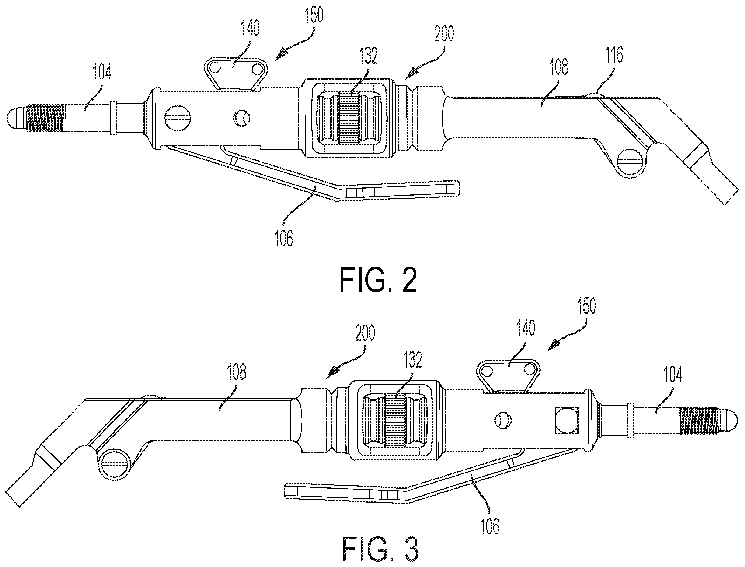

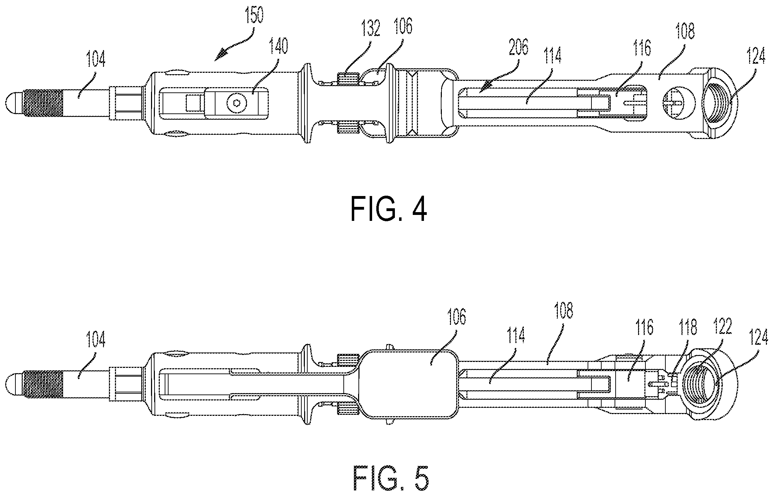

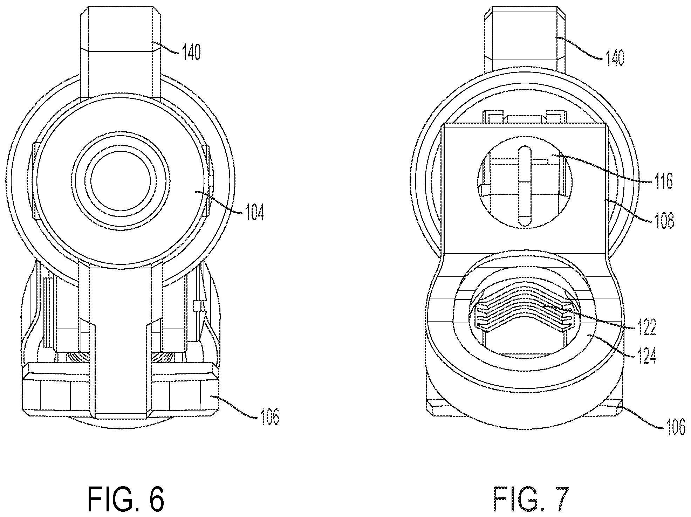

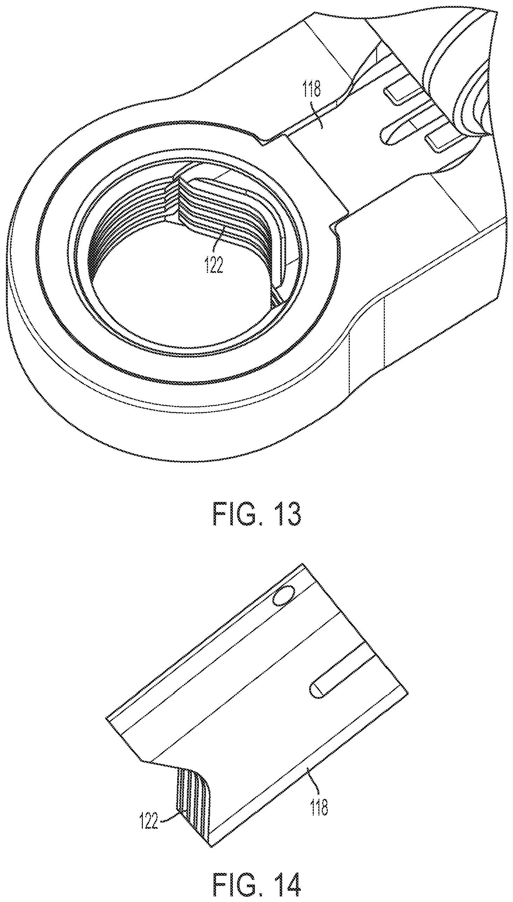

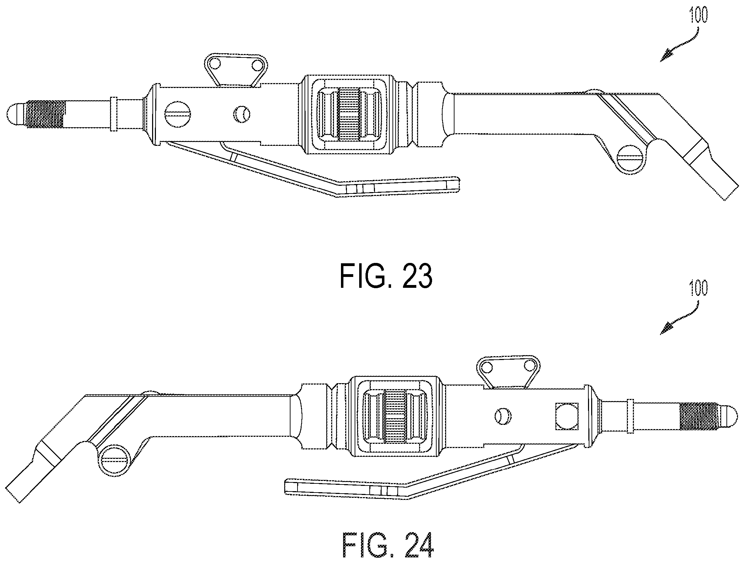

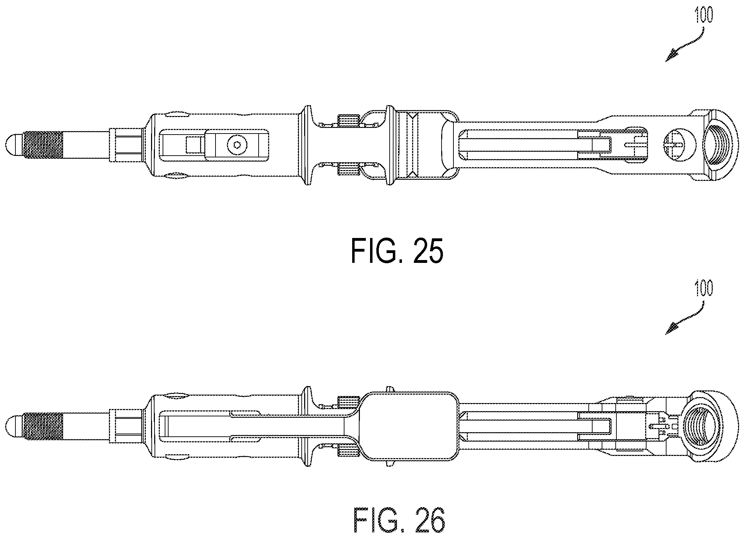

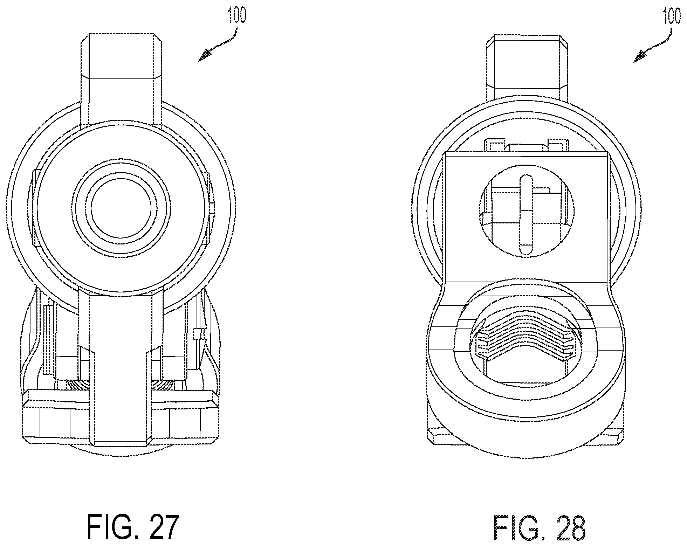

The subject disclosure provides an implant extractor medical device for use in orthopedic surgeries. In accordance with an exemplary embodiment, the implant extractor is a hip stem implant extractor 100. FIGS. 1-28 show the construction of the exemplary hip stem implant extractor 100 constructed in accordance with the subject disclosure.

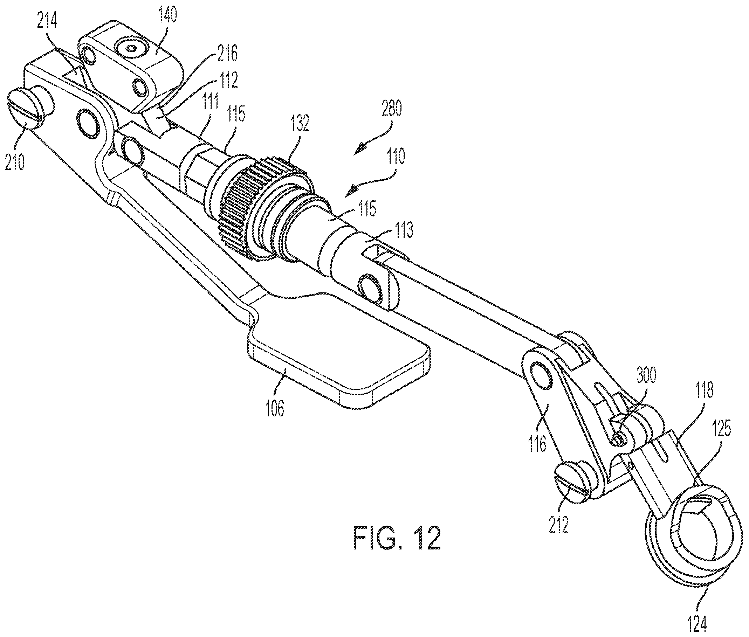

The hip stem implant extractor 100 includes a linkage assembly 280 (FIG. 12) operably secured within an elongate housing 200. The linkage assembly 280 is coupled to a jaw 118 which is movable between first and second positions under the influence of a cam lock 320 having pivotable handle 106. As further discussed below, the jaw is configured for clamping e.g., a trunnion (not shown).

The elongate housing 200 has a proximal end 202 and opposite distal end 204. The elongate housing includes a proximal body member 102 joined to a distal body member 108. A continuous recess or elongate continuous recess 206 extends through the proximal body member and the distal body member to operably receive the linkage assembly 280 therein.

The proximal body member 102 includes a tool connector 104 adapted for connection to an extraction tool, e.g., a T-handle or C-frame, and the like. In the present exemplary embodiment, the tool connector is a threaded tool connector.

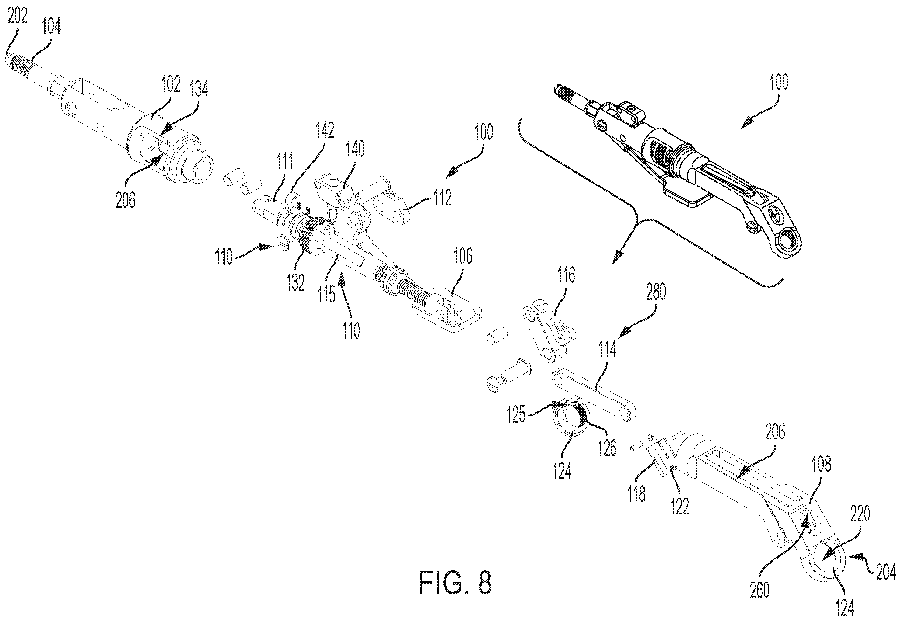

The distal body member 108 is configured as best shown in FIG. 1 and includes a main body portion and a bent arm portion that extends from the main body portion about 40-50 degrees, including 35, 45, and 55 degrees, from a longitudinal axis of the main body portion. The bent arm portion includes a through hole or opening 220 for receiving an implant therein e.g., a trunnion of a hip stem implant. The continuous recess 206 extends to the opening 220. The opening can include an insert 124 detachably secured thereto. The insert 124 can include grip enhancing features e.g., teeth 122, ridges or the like, about its inner facing surfaces. The insert 124 also includes an opening 125 (FIG. 8) in a circumferential wall thereof to receive the distal end of the jaw 118. When the insert is assembled to the opening 220, the opening 125 is aligned with the continuous recess 206 and in fluid communication therewith. The insert 124 is a removable insert detachably secured to the housing 200 at the distal end 204, the removable insert 124 has a surface for engaging the implant and an opening 125 for receiving the jaw 118 therethrough.

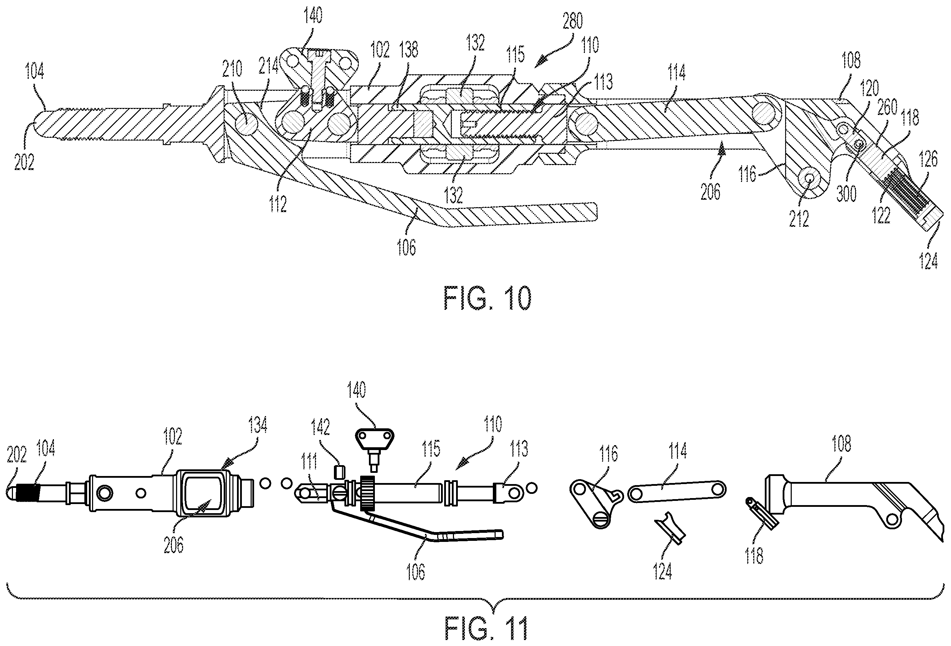

As best shown in FIGS. 10 & 12, the linkage assembly 280 includes a proximal link 112 having a cam race 216. The proximal link 112 is pivotally secured to the cam lock 320 having an elongate handle 106 and a cam 214 that operably engages the cam race 216. The elongate handle 106 is operatively connected to the proximal link 112 about its proximal end, as best shown in FIG. 10. The opposite distal end of the proximal link 112 is pivotally secured to a proximal link connector 111. The opposite end of the proximal link connector operably engages a rotating shaft 115 that threadably engages an intermediate link connector 113.

The handle 106 is pivotably connected to the housing 200 and operatively engaged with the linkage assembly 280. The handle 106 is pivotably connected to the housing and operatively engaged with the rod assembly 110. Placement of the handle 106 in a first position causes the jaw 118 to move to a disengagement state, and placement of the handle 106 in a second position causes the jaw 118 to move to an engagement state

The cam lock 320 is configured to lock the linkage assembly in a fixed position. The cam lock 320 is configured to apply a locking force and lock the linkage assembly 280 in a fixed position.

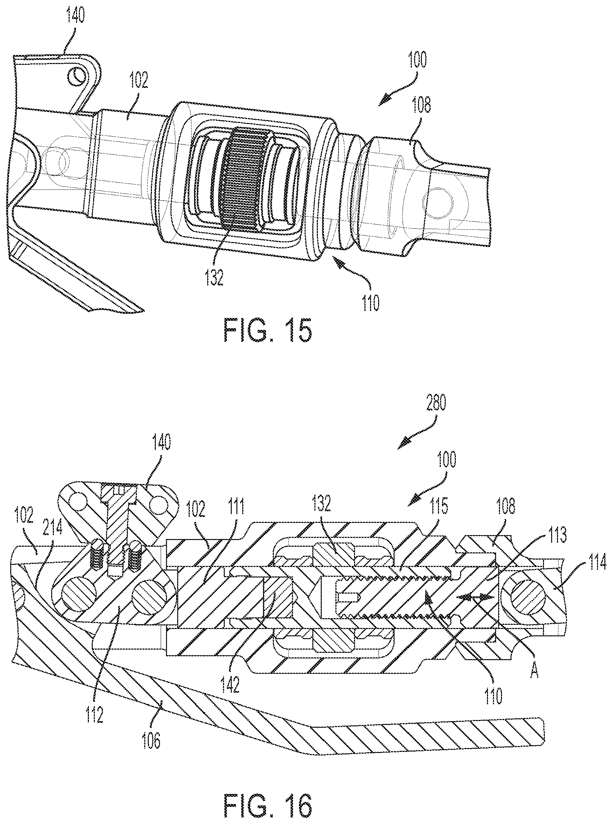

As best shown in FIGS. 15 & 16, the linkage assembly 280 includes a rod assembly 110 that is situated in part in the proximal body member 102 and in part in the distal body member 108. The rod assembly is configured as a telescopic rod assembly. A proximal end of the telescopic rod assembly 110 includes the proximal link connector 111 that is pivotably connected to a distal end of the proximal link 112. A distal end of the telescopic rod assembly 110 includes the intermediate link connector 113 that is pivotably connected to a proximal end of an intermediate link 114. The intermediate link connector 113 includes a treaded shaft extending proximally for threadedly engaging the telescopic rod assembly 110. The telescopic rod assembly 110 comprises the central rotatable shaft 115 which is configured to rotate about the proximal link connector 111 and threadedly engage the intermediate link connector 113.

In sum, the linkage assembly 280 includes a rod assembly 110, a proximal link 112 operatively connected to the rod assembly 110, a proximal end of the rod assembly 110 pivotably connected to a distal end of the proximal link 112, an intermediate link 114 operatively connected to the rod assembly 110, and a bell link 116 operatively connected to the intermediate link 114 and the jaw 118. The rod assembly 110 comprises a proximal link connector 111 operatively connected to the proximal link 112, an intermediate link connector 113 operatively connected to the intermediate link 114, and a rotatable shaft 115 rotatable about the proximal link connector 111 and operatively engaged with the intermediate link connector 113.

A manually actuatable dial 132 operably engages the rotating shaft 115 to adjust the position of the intermediate link connector 113 to be infinitely positionable along the path defined by arrows A (FIG. 16) of its threaded engagement. As shown in FIG. 8, the rotating shaft 115 includes a plurality of lateral flats for operatively engaging corresponding lateral flats about inner surfaces of the dial 132. For example, the plurality of flats about the rotating shaft can be hexagonal in shape, while the corresponding lateral flats of the dial are correspondingly hexagonal in shape.

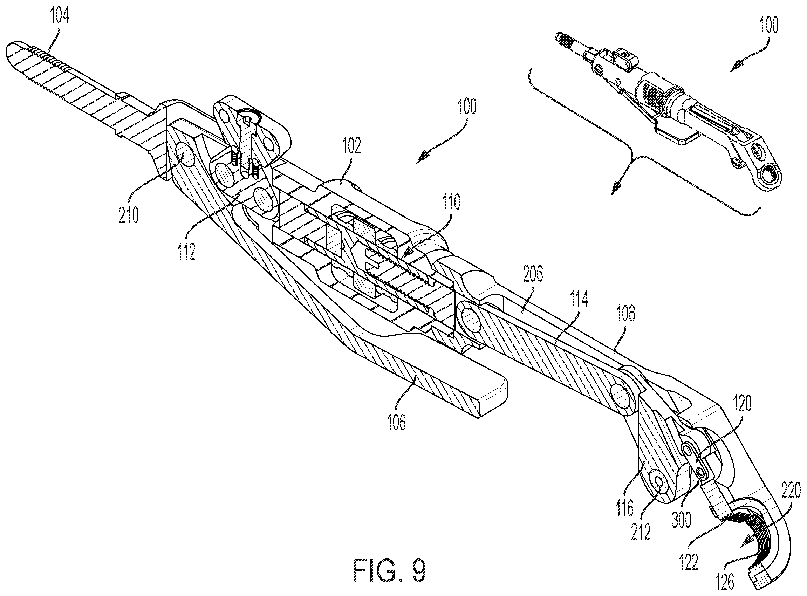

The opposite end of the link connector 113 is pivotally secured to one end of the elongate intermediate link 114. The opposite end or distal end of the intermediate link 114 is pivotally secured to a bell link 116. The jaw 118 is pivotally secured to an opposite side or distal end of the bell link 116 with jaw link 120 at pivot 300 (FIGS. 9, 10 and 12) and operably extends through insert 124. The bell link 116 can be configured e.g., as shown in FIG. 11, having a substantially triangular shape with three pivot points, a proximal pivot, a distal pivot, and a lateral pivot. The proximal pivot is pivotably engaged with the intermediate link and the distal pivot is pivotably engaged with the jaw, while the lateral pivot is pivotably engaged with the distal body member. Both the jaw 118 (similar to the insert 124) can include grip enhancing features, such as, teeth 126, ridges or the like for operably engaging an implant, e.g., a trunnion of a hip stem implant.

As best shown in FIGS. 1 & 10, the linkage assembly 280 of FIG. 12 is operably secured within the continuous recess 206 of the housing 200 with the handle pivotally secured to the proximal body member 102 at pivot 210 and the bell link 116 pivotally secured to the distal body member 108 at pivot 212. The jaw 118 extends within and is slidable within the continuous recess 206 towards the proximal end 204. Openings within the proximal body member 102 provide access to the dial 132 whereby the position of the jaw 118 within the opening 220 can be selected by a user rotating the dial 132 about the rotating shaft one way or the other to allow for fine adjust of the amount of force applied to the jaw via the cam lock. That is, rotating the dial 132 urges the intermediate link connector 113 in one or the other direction along the continuous recess 206, in other words proximally or distally along the housing, thereby moving the intermediate link 114 to pivot the bell link 116 about pivot 212, and thereby cause the jaw 118 to move along the slot 260 (FIG. 8) in the distal body member in which the jaw 118 sits.

In sum, the dial 132 is operatively connected to the central rotatable shaft 115, whereby rotation of the dial 132 in a first direction operates to unthread the intermediate link connector 113 from the central rotatable shaft 115, and whereby rotation of the dial 132 in a second direction opposite the first direction operates to thread the intermediate link connector 113into the central rotatable shaft.

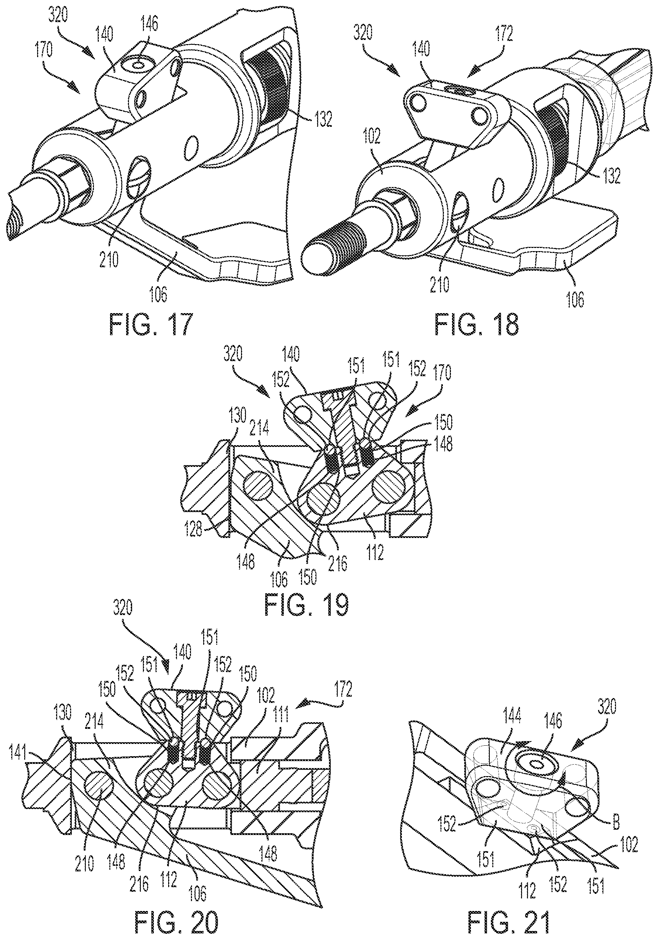

Additional clamping force (in addition to the force applied via the dial) can then be applied to the jaw 118 and thereon to an implant (e.g., a trunnion) in the opening by moving the handle from its open position 170 (FIGS. 17 & 19) to its closed position 172 (FIGS. 18 & 20). This causes the handle 106 to pivot about pivot 210 thereby urging cam 214 to operably engage cam race 216 of the proximal link 112 thereby urging the rotating shaft 115 toward the distal end 204 and causing the linkage assembly 280 to move the jaw 118 toward the opening 220.

More specifically, when the handle 106 is in the unlocked or fully opened position 170 (FIGS. 17 & 19), a first surface 128 at the proximal end of the handle 106 abuts a distally facing surface 130 of the proximal body member 102 and the cam 214 at the proximal end of the handle abuts a distally facing cam race 216 of the proximal link 112. With the handle unlocked, the user can adjust the length of the linkage assembly 280 to an appropriate length for receiving a subject implant in the opening to be gripped and to increase or decrease the preload or initial position (i.e., command position) of the jaw 118 within the housing or on an implant to be gripped. To do so, the user rotates the dial 132 having opening 134 corresponding to the circumferential shape of the central rotatable shaft 115, whereby the central rotatable shaft rotates in the same direction of rotation as the dial. Rotation of the central rotatable shaft in a first direction operates to unthread the intermediate link connector 113 from a distal internally threaded end of the central rotatable shaft. Simultaneously, a proximal cylindrical socket end of the central rotatable shaft rotates freely about a distal end of the proximal link connector 111. Likewise, rotation of the central rotatable shaft in the second direction operates to thread the intermediate link connector 1133 to threads of the central rotatable shaft 115.

Rotation of the central rotatable shaft in the first direction moves the intermediate link 114, the bell link 116, the jaw link 120 and the jaw 118 in a distal direction to increase the preload on a subject implant in the opening 220 or adjust the position of the jaw within the continuous recess 206 of the housing. Conversely, rotation of the central rotatable shaft in a second direction opposite the first direction operates to thread the intermediate link connector 113 into the distal internally threaded end of the central rotatable shaft. Simultaneously, the proximal cylindrical socket end of the central rotatable shaft rotates freely about a distal end of the proximal link connector 111. The jaw link 120 links or couples the jaw to the bell link. In other words, rotation of the central rotatable shaft in the second direction moves the intermediate link 114, the bell link 116, the jaw link 120 and the jaw 118 in a proximal direction to decrease the preload on a subject implant within the opening 220 or adjust the position of the jaw within the continuous recess 206.

Once the linkage assembly 280 is properly adjusted to the receive the subject implant to be extracted and the preload to be applied thereto, the handle 106 is moved from the unlocked position 170 (FIGS. 17 & 19) to the locked position 172 (FIGS. 18 & 20), whereby the handle 106 moves the proximal link 112 in the direction of the telescopic rod assembly 110, thereby moving the telescopic rod assembly, the intermediate link 114, the bell link 116, the jaw link 120 and the jaw in a distal direction to urge the aforementioned engaging ridges, teeth, or the like 122, 126 to grip the subject implant within the opening 220.

At the same time and as best shown in FIG. 20, a second surface 141 at a proximal end of the handle 106 comes into abutment with a distally facing surface 130 of the proximal body member 102. This creates a slight over-bend of the proximal link 112 (FIG. 20) thereby locking the handle in a locked position which keeps the downstream links in a locked position and the jaw 118 in a clamped position about the trunnion. Preferably, the proximal cylindrical socket end of the central rotatable shaft 115 contains a compressible bushing 142 (FIG. 16). The compressible bushing 142 can be fabricated from a resilient polymer, such as, polyetherimide, or other generally stiff yet resilient polymers suitable for the intended purposes as a rigid compression member.

According to an aspect, and as best shown in FIGS. 1, 18 & 21, the hip stem implant extractor 100 further comprises a locking key 140 connected to the proximal link 112 by a fastener 146 and rotatable about the fastener 146 in the direction of arrows B (FIG. 21) relative thereto between the unlocked position 170 (FIG. 17) and a locked position 172 (FIG. 18) extending substantially 90 degrees with respect to the unlocked position. As shown in FIGS. 19 & 20, the proximal link 112 may also include two sockets 150 within which are disposed springs 148 the upper ends of which biasingly support ball bearings 152. The spring-loaded ball bearings mate with two pairs of detents 151 to ensure that the locking key does not turn during extraction impacts. The mating ball bearings and detents also provide positive tactile feedback that the locking key 140 is firmly in its locked or unlocked position. In the unlocked position of the locking key, the handle 106 is free to move between its unlocked and locked positions. In the locked position of the locking key, the handle 106 is locked into its locked position whereby the jaw 118 is locked in its grip engaging position e.g., in gripping engagement with a hip stem implant trunnion, and extracting forces such as impacts may be applied to an extraction tool attached to the threaded connector 104 of the proximal body member 102.

The locking key 140 is operatively connected to the proximal link 112 and moveable between an unlocked position and a locked position. The locking key 140 locks the position of the proximal link 112 in a fixed position.

Having described the orientation and assembly of the various components of the hip stem implant extractor 100, its features become apparent. For example, the various links making up the linkage assembly 280 can be optimized to provide optimal clamping force by the jaw while minimizing the overall longitudinal length of the housing 100, thereby allowing a short hip stem implant extractor 100 e.g., a hip stem implant extractor that is shorter compared to conventional hip stem implant extractors.

Moreover, the linkage assembly's pivotal engagement of the jaw holds the jaw in a commanded position even if there is no force beyond gravity acting on the jaw. In contrast, gravity acting on conventional sliding trunnion engaging structures can freely slide the engaging structure into undesirable positions when a commanding force of the device is removed, or the device is in a non-locking state. Also, since the sliding jaw and the insert of the linkage assembly are detachably secured to the housing, they can be easily removed and replaced as needed in case these structures wear, without requiring the replacement of expensive surrounding components.

In other words, the hip stem implant extractor 100 provides a first engaging force or fine engaging force via the linkage assembly to position the jaw in the commanded position. The hip stem extractor also provides a second engaging force or locking force via the cam lock for securely engaging the jaw to the implant to be extracted.

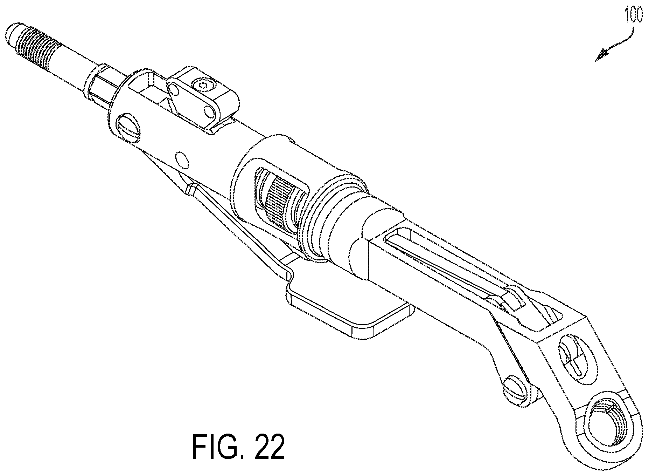

In accordance with another aspect of the subject disclosure, FIGS. 22-28 illustrate a design of the implant extractor. The relatively light intermittently broken surface shading depicts contour, not surface decoration. The broken surface shading lines shown throughout the figures depicts contours of the surgical tool. Various portions of the implant extractor design can be illustrated in broken lines to depict portions that form no part of the design.

## Claims

1. An implant extractor (100) for operably engaging a surgical implant, the implant extractor (100) having a housing (200) with a proximal end (202) configured for connection to an extraction tool, a distal end (204) for operably engaging the implant, the distal end has a continuous recess (206) extending therein, and a jaw (118) slidably received within and extending to the distal end (204) of the elongate housing (200)
an elongate recess (206) within the housing; and
a linkage assembly (280) within the elongate recess (206) of the housing (200), the linkage assembly (280) including a linkage (110, 114, 116, 120) pivotally secured to the jaw (118) such that operation of the linkage assembly (280) slides the jaw (118) towards and away from the distal end (204);
the linkage assembly (280) including:
a rod assembly (110);
a proximal link (112) operatively connected to the rod assembly (110);
a proximal end of the rod assembly (110) pivotably connected to a distal end of the proximal link (112);
an intermediate link (114) operatively connected to the rod assembly (110); and **characterized by** the linkage assembly (280) further including
a bell link (116) operatively connected to the intermediate link (114) and the jaw (118).

2. The implant extractor (100) of claim 1, wherein the elongate housing (200) includes an opening (220) at the distal end configured for receiving the implant, or wherein the elongate recess (206) is in fluid communication with the opening (220).

3. The implant extractor (100) of any one of claims 1 to 2, further comprising a handle (106) pivotably connected to the housing (200) and operatively engaged with the linkage assembly (280).

4. The implant extractor (100) of any one of claims 1 to 3, wherein the jaw (118) is detachably secured within the elongate housing (200).

5. The implant extractor (100) of any one of claims 1 to 4, further comprising a removable insert (124) detachably secured to the housing (200) at the distal end (204), the removable insert (124) having a surface for engaging the implant and an opening (125) for receiving the jaw therethrough.

6. The implant extractor (100) of any one of claims 1 to 5, further comprising a cam lock (320) configured to lock the linkage assembly in a fixed position, or a cam lock (320) configured to apply a locking force and lock the linkage assembly in a fixed position.

7. The implant extractor (100) of claim 1, further comprising a handle (106) pivotably connected to the housing (200) and operatively engaged with the rod assembly (110).

8. The implant extractor (100) of claim 7, wherein placement of the handle (106) in a first position causes the jaw (118) to move to a disengagement state, and placement of the handle (106) in a second position causes the jaw (118) to move to an engagement state.

9. The implant extractor (100) of any one of claims 1 to 8, wherein the rod assembly (110) is a telescopic rod assembly.

10. The implant extractor (100) of any one of claims 1 to 8, wherein the rod assembly (110) comprises:
a proximal link connector (111) operatively connected to the proximal link (112);
an intermediate link connector (113) operatively connected to the intermediate link (114); and
a rotatable shaft (115) rotatable about the proximal link connector (111) and operatively engaged with the intermediate link connector (113).

11. The implant extractor (100) of claim 10, further comprising a dial (132) operatively connected to the central rotatable shaft (115), whereby rotation of the dial (132) in a first direction operates to unthread the intermediate link connector (113) from the central rotatable shaft (115), and whereby rotation of the dial (132) in a second direction opposite the first direction operates to thread the intermediate link connector (113) into the central rotatable shaft (115).

12. The implant extractor (100) of any one of claims 1 to 11, further comprising a jaw link coupling (120) the bell link (116) to the jaw (118).

13. The implant extractor (100) of any one of claims 1 to 12, further comprising a locking key (140) operatively connected to the proximal link (112) and moveable between an unlocked position and a locked position.

14. The implant extractor (100) of claim 13, wherein the locking key (140) locks the position of the proximal link (112) in a fixed position.

## Patentansprüche

1. Implantatextraktor (100) zum wirksamen Eingreifen in ein chirurgisches Implantat, wobei der Implantatextraktor (100) ein Gehäuse (200) mit einem proximalen Ende (202), das für die Verbindung mit einem Extraktionswerkzeug konfiguriert ist, und einem distalen Ende (204) zum wirksamen Eingreifen in das Implantat aufweist, wobei das distale Ende eine sich darin erstreckende durchgehende Aussparung (206) aufweist, und eine Backe (118), die verschiebbar darin aufgenommen ist und sich bis zu dem distalen Ende (204) des länglichen Gehäuses (200) erstreckt;
eine längliche Aussparung (206) innerhalb des Gehäuses; und
eine Gestängebaugruppe (280) innerhalb der länglichen Aussparung (206) des Gehäuses (200), wobei die Gestängebaugruppe (280) ein Gestänge (110, 114, 116, 120) einschließt, das schwenkbar an der Backe (118) befestigt ist, sodass eine Betätigung der Gestängebaugruppe (280) die Backe (118) in Richtung des distalen Endes (204) und von diesem weg verschiebt;
die Gestängebaugruppe (280), einschließend:
eine Stangenbaugruppe (110);
ein proximales Glied (112), das funktionell mit der Stangenbaugruppe (110) verbunden ist;
ein proximales Ende der Stangenbaugruppe (110), das schwenkbar mit einem distalen Ende des proximalen Glieds (112) verbunden ist;
ein Zwischenglied (114), das funktionell mit der Stangenbaugruppe (110) verbunden ist: und **gekennzeichnet durch** die Gestängebaugruppe (280), ferner einschließend
ein Glockenglied (116), das funktionell mit dem Zwischenglied (114) und der Backe (118) verbunden ist.

2. Implantatextraktor (100) nach Anspruch 1, wobei das längliche Gehäuse (200) eine Öffnung (220) am distalen Ende einschließt, die zur Aufnahme des Implantats konfiguriert ist, oder wobei die längliche Aussparung (206) in Fluidverbindung mit der Öffnung (220) steht.

3. Implantatextraktor (100) nach einem der Ansprüche 1 bis 2, ferner umfassend einen Griff (106), der schwenkbar mit dem Gehäuse (200) verbunden ist und funktionell mit der Gestängebaugruppe (280) in Eingriff steht.

4. Implantatextraktor (100) nach einem der Ansprüche 1 bis 3, wobei die Backe (118) in dem länglichen Gehäuse (200) lösbar befestigt ist.

5. Implantatextraktor (100) nach einem der Ansprüche 1 bis 4, ferner umfassend einen entfernbaren Einsatz (124), der lösbar an dem Gehäuse (200) an dem distalen Ende (204) befestigt ist, wobei der entfernbare Einsatz (124) eine Oberfläche zum Eingreifen in das Implantat und eine Öffnung (125) zur Aufnahme der Backe dorthindurch aufweist.

6. Implantatextraktor (100) nach einem der Ansprüche 1 bis 5, ferner umfassend eine Nockenverriegelung (320), die konfiguriert ist, um die Gestängebaugruppe in einer festen Position zu verriegeln, oder eine Nockenverriegelung (320), die konfiguriert ist, um eine Verriegelungskraft auszuüben und die Gestängebaugruppe in einer festen Position zu verriegeln.

7. Implantatextraktor (100) nach Anspruch 1, ferner umfassend einen Griff (106), der schwenkbar mit dem Gehäuse (200) verbunden ist und funktionell mit der Stangenbaugruppe (110) in Eingriff steht.

8. Implantatextraktor (100) nach Anspruch 7, wobei die Positionierung des Griffs (106) in einer ersten Position bewirkt, dass sich die Backe (118) in einen Außereingriffszustand bewegt, und die Positionierung des Griffs (106) in einer zweiten Position bewirkt, dass sich die Backe (118) in einen Eingriffszustand bewegt.

9. Implantatextraktor (100) nach einem der Ansprüche 1 bis 8, wobei die Stangenbaugruppe (110) eine Teleskopstangenbaugruppe ist.

10. Implantatextraktor (100) nach einem der Ansprüche 1 bis 8, wobei die Stangenbaugruppe (110) Folgendes umfasst:
einen proximalen Gliedverbinder (111), der funktionell mit dem proximalen Glied
(112) verbunden ist;
einen Zwischengliedverbinder (113), der funktionell mit dem Zwischenglied (114) verbunden ist; und
eine drehbare Welle (115), die um den proximalen Gliedverbinder (111) drehbar ist und funktionell mit dem Zwischengliedverbinder (113) in Eingriff steht.

11. Implantatextraktor (100) nach Anspruch 10, ferner umfassend ein Einstellrad (132), das funktionell mit der zentralen drehbaren Welle (115) verbunden ist, wobei eine Drehung des Einstellrads (132) in eine erste Richtung bewirkt, dass der Zwischengliedverbinder (113) von der zentralen drehbaren Welle (115) abgeschraubt wird, und wobei eine Drehung des Einstellrads (132) in eine zweite Richtung, die der ersten Richtung entgegengesetzt ist, bewirkt, dass der Zwischengliedverbinder (113) in die zentrale drehbare Welle (115) eingeschraubt wird.

12. Implantatextraktor (100) nach einem der Ansprüche 1 bis 11, ferner umfassend eine Backengliedkopplung (120), die das Glockenglied (116) mit der Backe (118) koppelt.

13. Implantatextraktor (100) nach einem der Ansprüche 1 bis 12, ferner umfassend einen Verriegelungskeil (140), der funktionell mit dem proximalen Glied (112) verbunden und zwischen einer entriegelten Position und einer verriegelten Position bewegbar ist.

14. Implantatextraktor (100) nach Anspruch 13, wobei der Verriegelungskeil (140) die Position des proximalen Glieds (112) in einer festen Position verriegelt.

## Revendications

1. Extracteur d'implant (100) pour venir en prise de manière fonctionnelle avec un implant chirurgical, l'extracteur d'implant (100) ayant un logement (200) avec une extrémité proximale (202) conçue pour une connexion à un outil d'extraction, une extrémité distale (204) pour venir en prise de manière fonctionnelle avec l'implant, l'extrémité distale a un évidement continu (206) s'étendant à l'intérieur de celle-ci, et une mâchoire (118) reçue de manière coulissante à l'intérieur et s'étendant jusqu'à l'extrémité distale (204) du logement allongé (200)
un évidement allongé (206) dans le boîtier ; et
un ensemble de liaisons (280) à l'intérieur de l'évidement allongé (206) du boîtier (200), l'ensemble de liaisons (280) comportant une liaison (110, 114, 116, 120) fixée de manière pivotante à la mâchoire (118) de telle sorte que l'actionnement de l'ensemble de liaisons (280) fait coulisser la mâchoire (118) vers et à l'écart de l'extrémité distale (204) ;
l'ensemble de liaison (280) comportant :
un ensemble de tige (110) ;
un lien proximal (112) relié de manière fonctionnelle à l'ensemble de tige (110) ;
une extrémité proximale de l'ensemble de tige (110) reliée de manière pivotante à une extrémité distale de la liaison proximale (112) ;
un élément de liaison intermédiaire (114) relié de manière fonctionnelle à l'ensemble de tige (110) ; et **caractérisé par** l'ensemble de liaisons (280) comportant en outre
un lien coudé (116) relié de manière fonctionnelle au lien intermédiaire (114) et à la mâchoire (118).

2. Extracteur d'implant (100) selon la revendication 1, dans lequel le boîtier allongé (200) comporte une ouverture (220) à l'extrémité distale conçue pour recevoir l'implant, ou dans lequel l'évidement allongé (206) est en communication fluidique avec l'ouverture (220).

3. Extracteur d'implant (100) selon l'une quelconque des revendications 1 à 2, comprenant en outre une poignée (106) reliée de manière pivotante au boîtier (200) et en prise fonctionnelle avec l'ensemble de liaisons (280).

4. Extracteur d'implant (100) selon l'une quelconque des revendications 1 à 3, dans lequel la mâchoire (118) est fixée de manière amovible à l'intérieur du boîtier allongé (200).

5. Extracteur d'implant (100) selon l'une quelconque des revendications 1 à 4, comprenant en outre un insert amovible (124) fixé de manière détachable au boîtier (200) au niveau de l'extrémité distale (204), l'insert amovible (124) ayant une surface pour venir en prise avec l'implant et une ouverture (125) pour recevoir la mâchoire à travers celle-ci.

6. Extracteur d'implant (100) selon l'une quelconque des revendications 1 à 5, comprenant en outre un verrou à came (320) conçu pour verrouiller l'ensemble de liaisons dans une position fixe, ou un verrou à came (320) conçu pour appliquer une force de verrouillage et verrouiller l'ensemble de liaisons dans une position fixe.

7. Extracteur d'implant (100) selon la revendication 1, comprenant en outre une poignée (106) reliée de manière pivotante au boîtier (200) et en prise fonctionnelle avec l'ensemble tige (110).

8. Extracteur d'implant (100) selon la revendication 7, dans lequel le placement de la poignée (106) dans une première position amène la mâchoire (118) à se déplacer vers un état de désengagement, et le placement de la poignée (106) dans une seconde position amène la mâchoire (118) à se déplacer vers un état d'engagement.

9. Extracteur d'implant (100) selon l'une quelconque des revendications 1 à 8, dans lequel l'ensemble de tige (110) est un ensemble de tige télescopique.

10. Extracteur d'implant (100) selon l'une quelconque des revendications 1 à 8, dans lequel l'ensemble de tige (110) comprend :
un connecteur de liaison proximale (111) relié de manière fonctionnelle à la liaison proximale (112) ;
un connecteur de liaison intermédiaire (113) relié de manière fonctionnelle à la liaison intermédiaire (114) ; et
un arbre rotatif (115) pouvant tourner autour du connecteur de liaison proximal (111) et en prise fonctionnelle avec le connecteur de liaison intermédiaire (113).

11. Extracteur d'implant (100) selon la revendication 10, comprenant en outre une molette (132) reliée de manière fonctionnelle à l'arbre rotatif central (115), moyennant quoi la rotation de la molette (132) dans une première direction a pour effet de dévisser le connecteur de liaison intermédiaire (113) de l'arbre rotatif central (115), et moyennant quoi la rotation de la molette (132) dans une seconde direction opposée à la première direction a pour effet de visser le connecteur de liaison intermédiaire (113) dans l'arbre rotatif central (115).

12. Extracteur d'implant (100) selon l'une quelconque des revendications 1 à 11, comprenant en outre une liaison de mâchoire accouplant (120) la liaison de cloche (116) à la mâchoire (118).

13. Extracteur d'implant (100) selon l'une quelconque des revendications 1 à 12, comprenant en outre une clé de verrouillage (140) reliée de manière fonctionnelle à la liaison proximale (112) et mobile entre une position déverrouillée et une position verrouillée.

14. Extracteur d'implant (100) selon la revendication 13, dans lequel la clé de verrouillage (140) verrouille la position de la liaison proximale (112) dans une position fixe.
